# EUROPÄISCHE PATENTSCHRIFT

(11) **EP 1 095 027 B1**
(45) Veröffentlichungstag und Bekanntmachung des Hinweises auf die Patenterteilung: **15.06.2005**
(21) Anmeldenummer: 99934556.4
(22) Anmeldetag: 02.07.1999
(51) Int. Cl.: C07D 239/54, A01N 43/54, C07C 255/59, C07C 327/48, C07C 217/90, C07C 265/12, C07C 271/28

(54) **SUBSTITUIERTE PHENYLURACILE**
SUBSTITUTED PHENYL URACILS
PHENYLURACILE SUBSTITUE

(30) Priorität: 09.07.1998 DE 19830693; 23.11.1998 DE 19853864
(43) Veröffentlichungstag der Anmeldung: 02.05.2001
(73) Patentinhaber: Bayer CropScience AG, 40789 Monheim (DE)
(72) Erfinder: ANDREE, Roland, D-40764 Langenfeld (DE); DREWES, Mark, Wilhelm, D-40764 Langenfeld (DE); FEUCHT, Dieter, D-40789 Monheim (DE); PONTZEN, Rolf, D-42799 Leichlingen (DE); WETCHOLOWSKY, Ingo, CEP-13280-000 Vinhedo, SP (BR); SCHWARZ, Hans-Georg, D-40764 Langenfeld (DE)
(86) Internationale Anmeldenummer: PCT/EP1999/004585
(87) Internationale Veröffentlichungsnummer: WO 2000/002866

(56) Entgegenhaltungen:
- WO-A-93/14073
- WO-A-98/41093
- DE-A- 19 527 570

## Beschreibung

Die Erfindung betrifft neue substituierte Phenyluracile, Verfahren und neue Zwischenprodukte zu ihrer Herstellung sowie ihre Verwendung als Herbizide.

Bestimmte substituierte Aryluracile sind bereits aus der (Patent-)Literatur bekannt (vgl. EP-A-255047, EP-A-260621, EP-A-408382, EP-A-438209, EP-A-473551, EP-A-517181, EP-A-563384, WO-A-91/00278, WO-A-91/07393, WO-A-93/14073, WO-A-98/41093, US-A-4979982, US-A-5084084, US-A-5127935, US-A-5154755, US-A-5169430, US-A-5486610, US-A-5356863). Auch aus DE 195 27 570 A und WO 97/01541 A sind bereits substituierte Aryluracile mit herbizider Wirkung bekannt. Diese Verbindungen haben jedoch bisher keine besondere Bedeutung erlangt.

Es wurden nun neue substituierte Phenyluracile der allgemeinen Formel (IA) 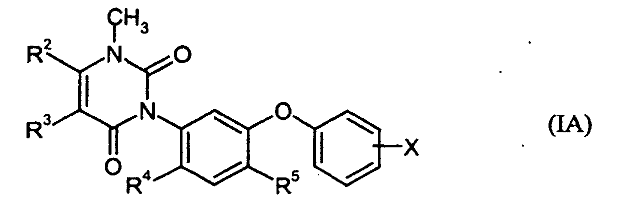 in welcher
- R²: für Trifluormethyl, Chlordifluormethyl, Difluormethyl oder Pentafluorethyl steht,
- R³: für Wasserstoff, Chlor oder Methyl steht,
- R⁴: für Wasserstoff, Fluor oder Chlor steht,
- R⁵: für Cyano oder Thiocarbamoyl steht, und
- X: für jeweils durch Carboxy, Methoxy, Ethoxy, n- oder i-Propoxy, Methoxycarbonyl, Ethoxycarbonyl, n- oder i-Propoxy-carbonyl, Allyloxycarbonyl, Propargyloxycarbonyl, 1-Buten-3-yl-oxy-carbonyl, 2-Buten-4-yl-oxycarbonyl, Propargyloxycarbonyl, 1-Butin-3-yl-oxy-carbonyl, 2-Butin-4-yl-oxy-carbonyl, Methylaminocarbonyl, Ethylaminocarbonyl, n- oder i-Propylamino-carbonyl, Dimethylaminocarbonyl, Diethylamino-carbonyl, Phenoxycarbonyl, Benzyloxycarbonyl, Phenylaminocarbonyl oder Benzylaminocarbonyl substituiertes Methyl, Ethyl, Methoxy, Ethoxy, Methylthio, Ethylthio, Methoxycarbonyl oder Ethoxycarbonyl, oder für durch Methoxycarbonyl oder Ethoxycarbonyl substiutiertes Ethenyl steht.
gefunden.

In den Definitionen sind die Kohlenwasserstoffketten, wie Alkyl - auch in Verbindung mit Heteroatomen, wie in Alkoxy - jeweils geradkettig oder verzweigt.

Soweit die erfindungsgemäßen Verbindungen der allgemeinen Formel (I) Substituenten mit asymmetrischen Kohlenstoffatomen enthalten, betrifft die Erfindung jeweils die R-Enantiomeren und die S-Enantiomeren sowie beliebige Mischungen dieser Enantiomeren, insbesondere die Racemate.

Eine ganz besonders bevorzugte Gruppe sind diejenigen Verbindungen der Formel (IA), bei welchen
- R²: für Trifluormethyl,
- R³: für Wasserstoff steht,
- R⁴: für Fluor steht,
- R⁵: für Cyano steht, und
- X: für 4-Carboxymethoxy, 4-Methoxycarbonylmethoxy, 4-Ethoxycarbonylmethoxy, 4-n-Propoxycarbonylmethoxy, 4-i-Propoxycarbonylmethoxy, 4-(1-Carboxy-ethoxy), 4-(1-(Methoxycarbonyl)-ethoxy), 4-(1-(Ethoxycarbonyl)-ethoxy), 4-(1-(n-Propoxycarbonyl)-ethoxy), 4-(1-(i-Propoxycarbonyl)-ethoxy), 4-(Allyloxycarbonylmethoxy), 4-(1-(Allyloxycarbonyl)-ethoxy), 4-(Propargyloxycarbonylmethoxy), 4-(1-(Propargyloxycarbanyl)-ethoxy), 4-(Benzyloxycarbonylmethoxy), 4-(1-(Benzyloxycarbonyl)-ethoxy), 4-(Aminocarbonylmethoxy), 4-(Methylaminocarbonylmethoxy), 4-(Ethylaminocarbonylmethoxy), 4-(n-Propylaminocarbonylmethoxy), 4-(i-Propyl-aminocarbonylmethoxy), 4-(Dimethylaminocarbonylmethoxy), 4-(1-(Methylaminocarbonyl)-ethoxy), 4-(1-(Ethylaminocarbonyl)-ethoxy), 4-(1-(n-Propylaminocarbonyl)-ethoxy), 4-(1-(i-Propylaminocarbonyl)-ethoxy), 4-(1-(Dimethylaminocarbonyl)-ethoxy), 4-(2-Methoxycarbonyl-ethenyl), 4-(2-Ethoxycarbonyl-ethenyl) steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Beispiele für die erfindungsgemäßen Verbindungen der allgemeinen Formel (I) sind in den nachstehenden Gruppen aufgeführt. 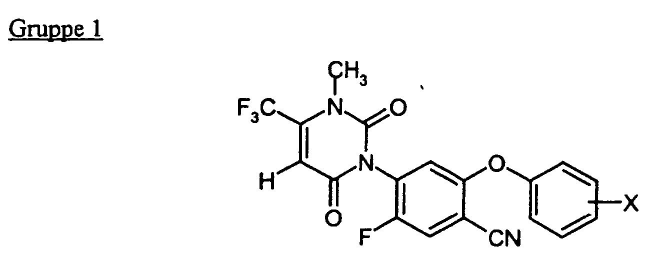
- X: hat dabei die in der nachstehenden Auflistung angegebenen Bedeutungen: 4-Carboxymethoxy, 4-Methoxycarbonylmethoxy, 4-Ethoxycarbonylmethoxy, 4-n-Propoxycarbonylmethoxy, 4-i-Propoxycarbonylmethoxy, 4-(1-Carboxy-ethoxy), 4-(1-(Methoxycarbonyl)-ethoxy), 4-(1-(Ethoxycarbonyl)-ethoxy), 4-(1-(n-Propoxycarbonyl)-ethoxy), 4-(1-(i-Propoxycarbonyl)-ethoxy), 4-(Allyloxycarbonylmethoxy), 4-(1-(Allyloxycarbonyl)-ethoxy), 4-(Propargyloxycarbonylmethoxy), 4-(1-(Propargyloxycarbonyl)-ethoxy), 4-(Benzyloxycarbonylmethoxy), 4-(1-(Benzyloxycarbonyl)-ethoxy), 4-(Aminocarbonylmethoxy), 4-(Methylaminocarbonylmethoxy), 4-(Ethylaminocarbonylmethoxy), 4-(n-Propylaminocarbonylmethoxy), 4-(i-Propylaminocarbonylmethoxy), 4-(Dimethylaminocarbonylmethoxy), 4-(1-(Methylaminocarbonyl)-ethoxy), 4-(1-(Ethylaminocarbonyl)-ethoxy), 4-(1-(n-Propylaminocarbonyl)-ethoxy), 4-(1-(i-Propylaminocarbonyl)-ethoxy), 4-(1-(Dimethylaminocarbonyl)-ethoxy), 4-(2-Methoxycarbonyl-ethenyl), 4-(2-Ethoxycarbonyl-ethenyl).

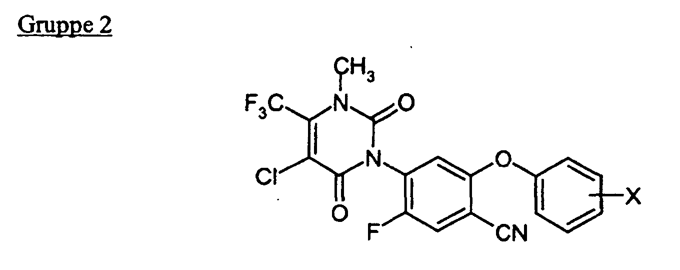

- X: hat dabei die oben in Gruppe 1 angegebenen Bedeutungen.

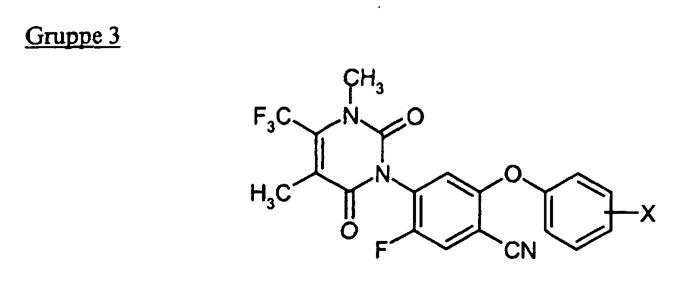

- X: hat dabei die oben in Gruppe 1 angegebenen Bedeutungen.

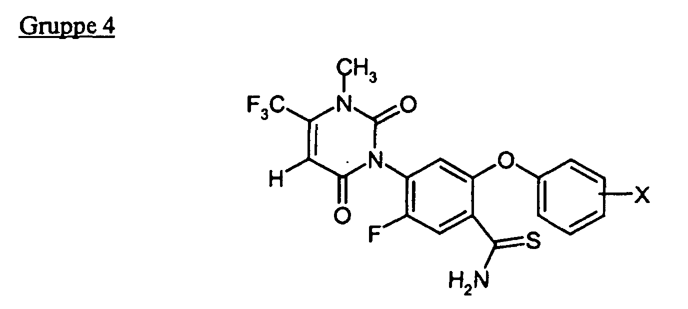

- X: hat dabei die oben in Gruppe 1 angegebenen Bedeutungen.

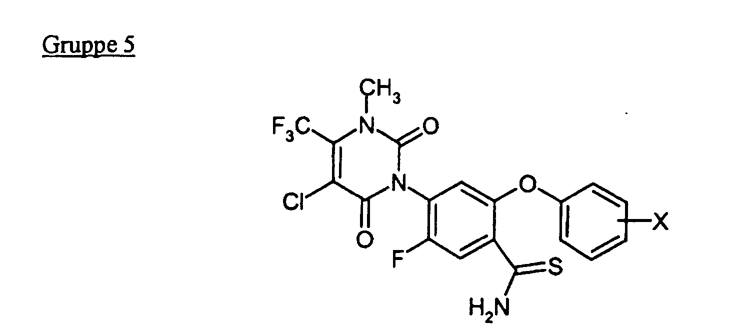

- X: hat dabei die oben in Gruppe 1 angegebenen Bedeutungen.

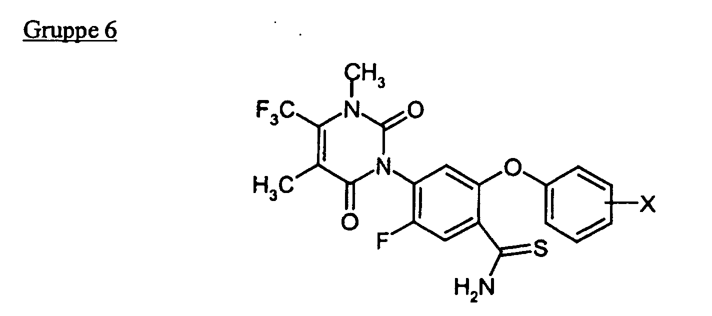

- X: hat dabei die oben in Gruppe 1 angegebenen Bedeutungen.

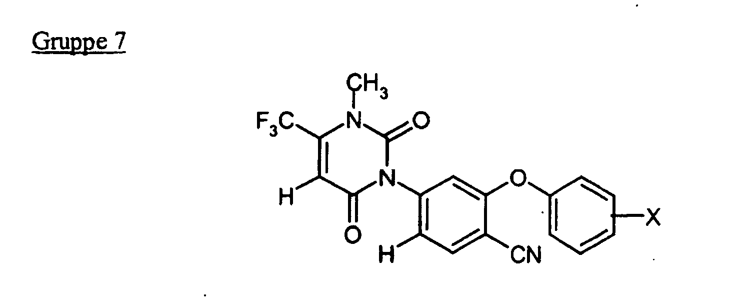

- X: hat dabei die oben in Gruppe 1 angegebenen Bedeutungen.

Die neuen substituierten Phenyluracile der allgemeinen Formel (IA) weisen interessante biologische Eigenschaften auf. Sie zeichnen sich insbesondere durch starke herbizide Wirksamkeit aus.

Man erhält die neuen substituierten Phenyluracile der allgemeinen Formel (IA), wenn man
(a) Halogenophenyluracile der allgemeinen Formel (II) 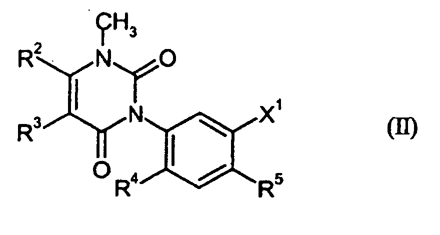 in welcher
   - R², R³, R⁴ und R⁵: die oben angegebene Bedeutung haben und
   - X¹: für Halogen steht,
   mit Arylverbindungen der allgemeinen Formel (III) 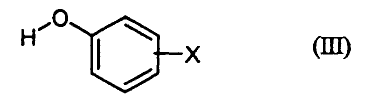 in welcher
   - X: die oben angegebene Bedeutung hat,

   - oder mit Metallsalzen von Verbindungen der allgemeinen Formel (III) -

   gegebenenfalls in Gegenwart eines Reaktionshilfsmittels und gegebenenfalls in Gegenwart eines Verdünnungsmittels umsetzt,
   oder wenn man
(b) Aminoalkensäureester der allgemeinen Formel (IV) 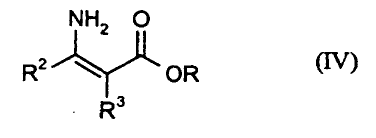 in welcher
   - R² und R³: die oben angegebene Bedeutung haben und
   - R: für Alkyl, Aryl oder Arylalkyl steht,
   mit Arylisocyanaten der allgemeinen Formel (V) 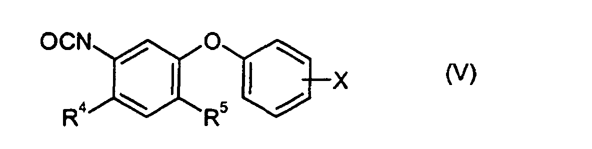 in welcher
   - R⁴, R⁵ und X: die oben angegebene Bedeutung haben,
   oder mit Arylurethanen (Arylcarbamaten) der allgemeinen Formel (VI) 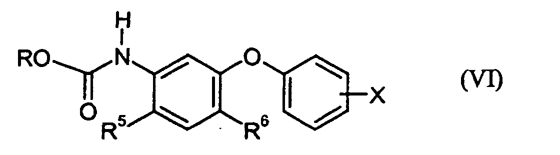 in welcher
   - R⁵, R⁶ und X: die oben angegebene Bedeutung haben und
   - R: für Alkyl, Aryl oder Arylalkyl steht,
   gegebenenfalls in Gegenwart eines Reaktionshilfsmittels und gegebenenfalls in Gegenwart eines Verdünnungsmittels umsetzt,
   oder wenn man
(c) N-Aryl-1-alkoxycarbonylamino-maleinimide der allgemeinen Formel (VII) 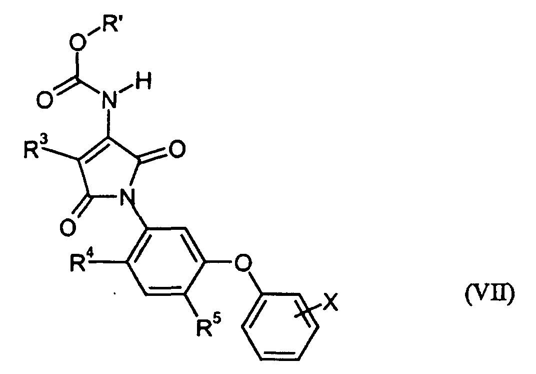 in welcher
   - R³, R⁴, R⁵ und X: die oben angegebene Bedeutung haben und
   - R': für Alkyl steht,
   mit einem Metallhydroxid in Gegenwart von Wasser und gegebenenfalls in Gegenwart eines organischen Lösungsmittels umsetzt,
   oder wenn man
(d) substituierte Phenyluracile der allgemeinen Formel (Ia) 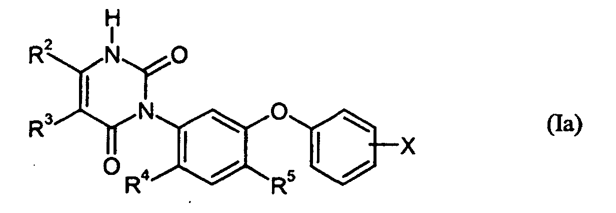
in welcher
- R², R³, R⁴, R⁵ und X: die oben angegebene Bedeutung haben,
mit 1-Aminooxy-2,4-dinitro-benzol oder mit Alkylierungsmitteln der allgemeinen Formel (VIII)

X²-A¹ (VIII)

in welcher
- A¹: für gegebenenfalls substituiertes Alkyl steht und
- X²: für Halogen oder die Gruppierung -O-SO₂-O-A¹ steht,
gegebenenfalls in Gegenwart eines Reaktionshilfsmittels und gegebenenfalls in Gegenwart eines Verdünnungsmittels umsetzt,
und gegebenenfalls im Anschluß daran im Rahmen der Substituentendefinition auf übliche Weise elektrophile oder nucleophile bzw. Oxidations- oder Reduktionsreaktionen durchführt.

Die Verbindungen der allgemeinen Formel (IA) können nach üblichen Methoden in andere Verbindungen der allgemeinen Formel (IA) gemäß obiger Definition umgewandelt werden, beispielsweise durch Veresterung bzw. Hydrolyse (z.B. X: OCH₂COOH → OCH₂COOC₂H₅, OCH(CH₃)COOCH₃ → OCH(CH₃)COOH), Umsetzung mit Dicyan bzw. Hydrogensulfid (z.B. R⁵: Br → CN, CN → CSNH₂, Umwandlung von Carboxyverbindungen in andere Carbonsäurederivate nach üblichen Methoden (z.B. R²: COOH → CN, CN → CSNH₂, COOH → COOCH₃, COOCH₃ → CONH₂); vgl. die Herstellungsbeispiele).

Verwendet man beispielsweise 1-[2-Chlor-4-trifluormethyl-5-(4-methoxycarbonylmethoxy-phenoxy)-phenyl]-4-difluormethyl-3,6-dihydro-2,6-dioxo-1 (2H)-pyrimidin und Methylbromid als Ausgangsstoffe, so kann der Reaktionsablauf beim erfindungsgemäßen Verfahren (d) durch das folgende Formelschema skizziert werden:

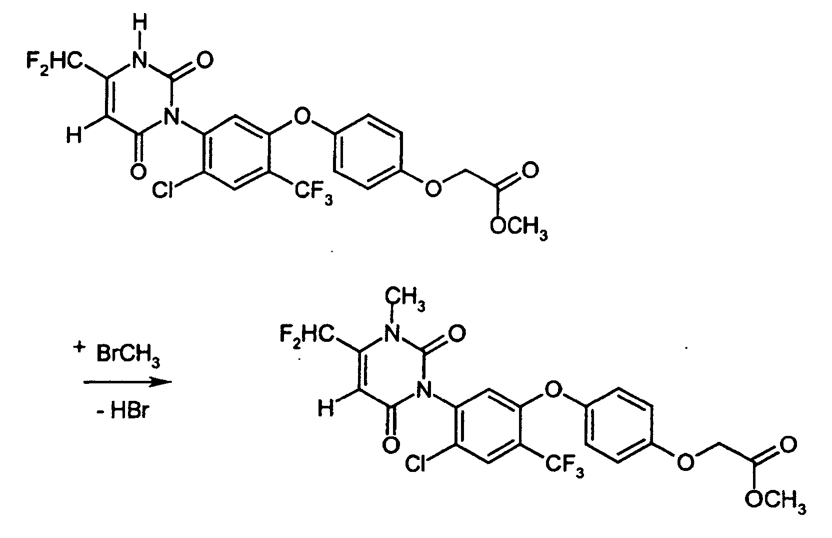

Die beim erfindungsgemäßen Verfahren (a) zur Herstellung von Verbindungen der Formel (IA) als Ausgangsstoffe zu verwendenden Halogenophenyluracile sind durch die Formel (II) allgemein definiert. In der Formel (II) haben R², R³, R⁴ und R⁵ insbesondere diejenigen Bedeutungen, die bereits oben im Zusammenhang mit der Beschreibung der erfindungsgemäßen Verbindungen der Formel (IA) als bevorzugt für R², R³, R⁴ und R⁵ angegeben wurden; X¹ steht vorzugsweise für Fluor oder Chlor, insbesondere für Fluor.

Die Ausgangsstoffe der allgemeinen Formel (II) sind bekannt und/oder können nach an sich bekannten Verfahren hergestellt werden (vgl. EP-A-648749).

Die beim erfindungsgemäßen Verfahren (a) weiter als Ausgangsstoffe zu verwendenden Arylverbindungen sind durch die Formel (III) allgemein definiert. In der Formel (III) hat X insbesondere diejenigen Bedeutungen, die bereits oben im Zusammenhang mit der Beschreibung der erfindungsgemäßen Verbindungen der Formel (IA) als bevorzugt, besonders bevorzugt oder ganz besonders bevorzugt für X angegeben wurden.

Die Ausgangsstoffe der allgemeinen Formel (III) sind bekannte organische Synthesechemikalien.

Die beim erfindungsgemäßen Verfahren (b) zur Herstellung von Verbindungen der allgemeinen Formel (IA) als Ausgangsstoffe zu verwendenden Aminoalkensäureester sind durch die Formel (IV) allgemein definiert. In der allgemeinen Formel (IV) haben R² und R³ insbesondere diejenigen Bedeutungen, die bereits oben im Zusammenhang mit der Beschreibung der erfindungsgemäßen Verbindungen der allgemeinen Formel (IA) als bevorzugt, besonders bevorzugt oder ganz besonders bevorzugt für R² und R³ angegeben worden sind; R steht vorzugsweise für C₁-C₄-Alkyl, Phenyl oder Benzyl, insbesondere für Methyl oder Ethyl.

Die Ausgangsstoffe der allgemeinen Formel (IV) sind bekannt und/oder können nach an sich bekannten Verfahren hergestellt werden (vgl. J. Heterocycl. Chem. 9 (1972), 513-522).

Die beim erfindungsgemäßen Verfahren (b) weiter als Ausgangsstoffe zu verwendenden Arylisocyanate sind durch die Formel (V) allgemein definiert. In der allgemeinen Formel (V) haben R⁴, R⁵ und X insbesondere diejenigen Bedeutungen, die bereits oben im Zusammenhang mit der Beschreibung der erfindungsgemäßen Verbindungen der allgemeinen Formel (IA) als bevorzugt, besonders bevorzugt oder ganz besonders bevorzugt für R⁴, R⁵ und X angegeben worden sind.

Die Ausgangsstoffe der allgemeinen Formel (V) sind noch nicht aus der Literatur bekannt; sie sind als neue Stoffe auch Gegenstand der vorliegenden Anmeldung.

Man erhält die neuen Arylisocyanate der allgemeinen Formel (V), wenn man Anilinderivate der allgemeinen Formel (IX) 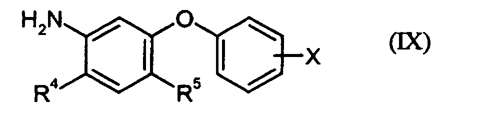 in welcher
- R⁴, R⁵ und X: die oben angegebene Bedeutung haben,
mit Phosgen in Gegenwart eines Verdünnungsmittels, wie z.B. Chlorbenzol, bei Temperaturen zwischen -20°C und +1 50°C umsetzt (vgl. z.B. auch EP-A-648749).

Die beim erfindungsgemäßen Verfahren (b) gegebenenfalls als Ausgangsstoffe zu verwendenden Arylurethane sind durch die Formel (VI) allgemein definiert. In der allgemeinen Formel (VI) haben R⁴, R⁵ und X insbesondere diejenigen Bedeutungen, die bereits oben im Zusammenhang mit der Beschreibung der erfindungsgemäßen Verbindungen der allgemeinen Formel (IA) als bevorzugt, besonders bevorzugt oder ganz besonders bevorzugt für R⁴, R⁵ und X angegeben worden sind; R steht vorzugsweise für C₁-C₄-Alkyl, Phenyl oder Benzyl, insbesondere für Methyl oder Ethyl.

Die Ausgangsstoffe der allgemeinen Formel (VI) sind noch nicht aus der Literatur bekannt; sie sind als neue Stoffe auch Gegenstand der vorliegenden Anmeldung.

Man erhält die neuen Arylurethane der allgemeinen Formel (VI), wenn man Anilinderivate der allgemeinen Formel (IX) 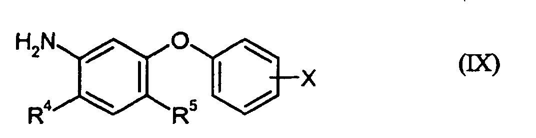 in welcher
- R⁴, R⁵ und X: die oben angegebene Bedeutung haben,
mit Chlorcarbonylverbindungen der allgemeinen Formel (X)

RO-CO-Cl (X)

in welcher
- R: die oben angegebene Bedeutung hat,
gegebenenfalls in Gegenwart eines Säureakzeptors, wie z.B. Pyridin, und gegebenenfalls in Gegenwart eines Verdünnungsmittels, wie z.B. Methylenchlorid, bei Temperaturen zwischen -20°C und +100°C umsetzt (vgl. die Herstellungsbeispiele).

Die als Vorprodukte benötigten Anilinderivate der allgemeinen Formel (IX) sind bekannt und/oder können nach an sich bekannten Verfahren hergestellt werden (vgl. Justus Liebigs Ann. Chem. 740 (1970), 169-179; US-A-3715395; US-A-3914418; DE-A-2748554; DE3736089).

Noch nicht bekannt sind die Anilinderivate der allgemeinen Formel (IXa) 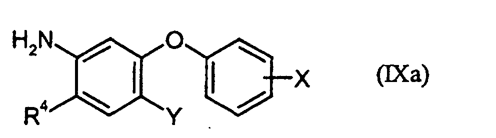 in welcher
- R⁴ und X: die oben angegebene Bedeutung haben und
- Y: für Cyano, Thiocarbamoyl oder Trifluormethyl steht.

Man erhält die Anilinderivate der allgemeinen Formel (IXa), wenn man Aniline der allgemeinen Formel (XI) 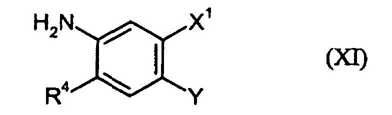 in welcher
- R⁴, X¹ und Y: die oben angegebene Bedeutung haben,
mit Arylverbindungen der allgemeinen Formel (III) 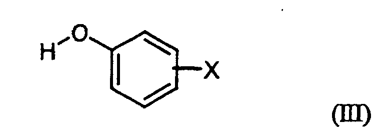 in welcher
- X: die oben angegebene Bedeutung hat,

- oder mit Metallsalzen von Verbindungen der allgemeinen Formel (III) - gegebenenfalls in Gegenwart eines Reaktionshilfsmittels, wie z.B. Natriumhydrid, und gegebenenfalls in Gegenwart eines Verdünnungsmittels, wie z.B. N-Methylpyrrolidon, bei Temperaturen zwischen 0°C und 150°C umsetzt (vgl. die Herstellungsbeispiele).

Die beim erfindungsgemäßen Verfahren (c) zur Herstellung von Verbindungen der allgemeinen Formel (IA) als Ausgangsstoffe zu verwendenden N-Aryl-1-alkoxycarbonylamino-maleinimide sind durch die Formel (VII) allgemein definiert. In der allgemeinen Formel (VII) haben R³, R⁴, R⁵ und X insbesondere diejenigen Bedeutungen, die bereits oben im Zusammenhang mit der Beschreibung der erfindungsgemäßen Verbindungen der allgemeinen Formel (IA) als bevorzugt, besonders bevorzugt oder ganz besonders bevorzugt für R³, R⁴, R⁵ und X angegeben worden sind; R' steht vorzugsweise für C₁-C₄-Alkyl, insbesondere für Methyl oder Ethyl.

Man erhält die N-Aryl-1-alkoxycarbonylamino-maleinimide der allgemeinen Formel (VII), wenn man (2,5-Dioxo-2,5-dihydro-furan-3-yl)-carbamidsäure-alkylester der allgemeinen Formel (XII) 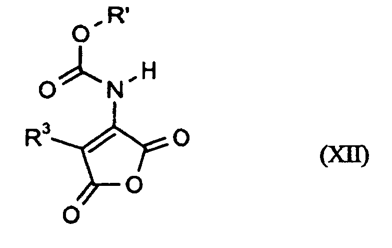 in welcher
- R³: die oben angegebene Bedeutung hat und
- R': für Alkyl (insbesondere für Methyl oder Ethyl) steht,
mit Anilinderivaten der allgemeinen Formel (IX) 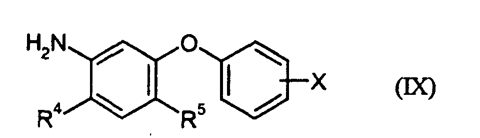 in welcher
- R⁴, R⁵ und X: die oben angegebene Bedeutung haben,
gegebenenfalls in Gegenwart eines Verdünnungsmittels, wie z.B. Essigsäure, bei Temperaturen zwischen 0°C und 200°C, vorzugsweise zwischen 50°C und 150°C umsetzt.

Die Vorprodukte der allgemeinen Formel (XII) sind bekannt und/oder können nach an sich bekannten Verfahren hergestellt werden (vgl. DE 19604229).

Die beim erfindungsgemäßen Verfahren (d) zur Herstellung von Verbindungen der Formel (IA) als Ausgangsstoffe zu verwendenden substituierten Phenyluracile sind durch die Formel (Ia) allgemein definiert. In der Formel (Ia) haben R², R³, R⁴, R⁵ und X insbesondere diejenigen Bedeutungen, die bereits oben im Zusammenhang mit der Beschreibung der erfindungsgemäßen Verbindungen der Formel (IA) als bevorzugt, besonders bevorzugt oder ganz besonders bevorzugt für R², R³, R⁴, R⁵ und X angegeben wurden.

Die Ausgangsstoffe der allgemeinen Formel (Ia) für Verfahren (b) sind als neue Stoffe auch Gegenstand der vorliegenden Anmeldung; sie können nach den erfindungsgemäßen Verfahren (a), (b) und (c) hergestellt werden.

Die beim erfindungsgemäßen Verfahren (d) weiter als Ausgangsstoffe zu verwendenden Alkylierungsmittel sind durch die Formel (VIII) allgemein definiert. In der Formel (VIII) stehen vorzugsweise A¹ für gegebenenfalls durch Cyano, Halogen oder C₁-C₄-Alkoxy substituiertes Alkyl mit 1 bis 4 Kohlenstoffatomen und X² für Chlor, Brom, Iod, Methylsulfonyloxy oder Ethylsulfonyloxy; insbesondere stehen A¹ für jeweils gegebenenfalls durch Cyano, Fluor, Chlor, Methoxy oder Ethoxy substituiertes Methyl, Ethyl, n- oder i-Propyl und X² für Chlor, Brom, Iod, Methylsulfonyloxy oder Ethylsulfonyloxy.

Die Ausgangsstoffe der Formel (VIII) sind bekannte organische Synthesechemikalien.

Die erfindungsgemäßen Verfahren zur Herstellung der Verbindungen der allgemeinen Formel (IA) werden vorzugsweise unter Verwendung von Verdünnungsmitteln durchgeführt. Als Verdünnungsmittel zur Durchführung der erfindungsgemäßen Verfahren (a), (b), (c) und (d) kommen neben Wasser vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutylketon; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Als Reaktionshilfsmittel für die erfindungsgemäßen Verfahren (a), (b), (c) und (d) kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calciumcarbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, -n- oder -i-propanolat, -n-, -i-, -soder -t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethylpyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8-Diazabicyclo[5,4,0]-undec-7-en (DBU).

Als weitere Reaktionshilfsmittel für die erfindungsgemäßen Verfahren kommen auch Phasentransfer-Katalysatoren in Betracht. Als Beispiele für solche Katalysatoren seien genannt:

Tetrabutylammonium-bromid, Tetrabutylammonium-chlorid, Tetraoctylammoniumchlorid, Tetrabutylammonium-hydrogensulfat, Methyl-trioctylammonium-chlorid, Hexadecyl-trimethylammonium-chlorid, Hexadecyl-trimethylammonium-bromid, Benzyl-trimethylammonium-chlorid, Benzyl-triethylammonium-chlorid, Benzyl-trimethylammonium-hydroxid, Benzyl-triethylammonium-hydroxid, Benzyl-tributylammonium-chlorid, Benzyl-tributylammonium-bromid, Tetrabutylphosphoniumbromid, Tetrabutylphosphonium-chlorid, Tributyl-hexadecylphosphonium-bromid, Butyl-triphenylphosphonium-chlorid, Ethyl-trioctylphosphonium-bromid, Tetraphenylphosphonium-bromid.

Die Reaktionstemperaturen können bei der Durchführung der erfindungsgemäßen Verfahren (a), (b), (c) und (d) in einem größeren Bereich variiert werden. Im allgemeinen arbeitet man bei Temperaturen zwischen 0°C und 150°C, vorzugsweise zwischen 10°C und 120°C.

Die erfindungsgemäßen Verfahren werden im allgemeinen unter Normaldruck durchgeführt. Es ist jedoch auch möglich, die erfindungsgemäßen Verfahren unter erhöhtem oder vermindertem Druck - im allgemeinen zwischen 0,1 bar und 10 bar - durchzuführen.

Zur Durchführung der erfindungsgemäßen Verfahren werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab. Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Acetochlor, Acifluorfen(-sodium), Aclonifen, Alachlor, Alloxydim(-sodium), Ametryne, Amidochlor, Amidosulfuron, Anilofos, Asutam, Atrazine, Azafenidin, Azimsulfuron, Benazolin(-ethyl), Benfuresate, Bensulfuron(-methyl), Bentazon, Benzofenap, Benzoylprop(-ethyl), Bialaphos, Bifenox, Bispyribac(-sodium), Bromobutide, Bromofenoxim, Bromoxynil, Butachlor, Butroxydim, Butylate, Cafenstrole, Caloxydim, Carbetamide, Carfentrazone(-ethyl), Chlomethoxyfen, Chloramben, Chloridazon, Chlorimuron(-ethyl), Chlornitrofen, Chlorsulfuron. Chlortoluron, Cinidon(-ethyl), Cinmethylin, Cinosulfuron, Clethodim, Clodinafop(-propargyl), Clomazone, Clomeprop, Clopyralid, Clopyrasulfuron(-methyl), Cloransulam(methyl), Cumyluron, Cyanazine, Cybutryne, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Diclosulam, Diethatyl(-ethyl), Difenzoquat, Diflufenican, Diflufenzopyr, Dimefuron, Dimepiperate, Dimethachlor, Dimethametryn, Dimethenamid, Dimexytlam, Dinitramine, Diphenamid, Diquat, Dithiopyr, Diuron, Dymron, Epoprodan, EPTC, Esprocarb, Ethalfluralin, Ethametsulfuron(-methyl), Ethofumesate, Ethoxyfen, Ethoxysulfuron, Etobenzanid, Fenoxaprop(-P-ethyl), Flamprop(-isopropyl), Flamprop(-isopropyl-L), Flamprop(-methyl), Flazasulfuron, F(uazifop(-Pbutyl), Fluazolate, Flucarbazone, Flufenacet, Flumetsulam, Flumiclorac(-pentyl), Flumioxazin, Flumipropyn, Flumetsulam, Fluometuron, Fluorochloridone, Fluoroglycofen(-ethyl), Flupoxam, Flupropacil, Flurpyrsulfuron(-methyl, -sodium), Flurenol(-butyl), Fluridone, Fluroxypyr(-meptyl), Flurprimidol, Flurtamone, Fluthiacet(-methyl), Fluthiamide, Fomesafen, Glufosinate(-ammonium), Glyphosate(isopropylammonium), Halosafen, Haloxyfop(-ethoxyethyl), Haloxyfop(-P-methyl), Hexazinone, Imazamethabenz(-methyl), Imazamethapyr, Imazamox, Imazapic, Imazapyr, Imazaquin, Imazethapyr, Imazosulfuron, Iodosulfuron, Ioxynil, Isopropalin, Isoproturon, Isouron, Isoxaben, Isoxachlortole, Isoxaflutole, Isoxapyrifop, Lactofen, Lenacil, Linuron, MCPA, MCPP, Mefenacet, Mesotrione, Metamitron, Metazachlor, Methabenzthiazuron, Metobenzuron, Metobromuron, (alpha-)Metolachlor, Metosulam, Metoxuron, Metribuzin, Metsulfuron(-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pentoxazone, Phenmedipham, Piperophos, Pretilachlor, Primisulfuron(-methyl), Prometryn, Propachlor, Propanil, Propaquizafop, Propisochlor, Propyzamide, Prosulfocarb, Prosulfuron, Pyraflufen(-ethyl), Pyrazolate, Pyrazosulfuron(-ethyl), Pyrazoxyfen, Pyribenzoxim, Pyributicarb, Pyridate, Pyriminobac(-methyl), Pyrithiobac(-sodium), Quinchlorac, Quinmerac, Quinoclamine, Quizalofop(-P-ethyl), Quizalofop(-P-tefuryl), Rimsulfuron, Sethoxydim, Simazine, Simetryn, Sulcotrione, Sulfentrazone, Sulfometuron(-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron(methyl), Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

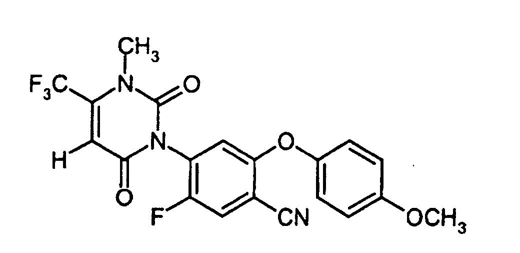

(Verfahren (b))

Eine Mischung aus 0,50 g (1,2 mMol) 4-(3,6-Dihydro-2,6-dioxo-4-trifluormethyl-1(2H)-pyrimidin-1-yl)-5-fluor-2-(4-methoxy-phenoxy)-benzonitril, 0,20 g (1,8 mMol) Dimethylsulfat, 0,30 g (2,4 mMol) Kaliumcarbonat und 100 ml Aceton wird 15 Stunden unter Rückfluß erhitzt und anschließend im Wasserstrahlvakuum eingeengt. Der Rückstand wird mit 50 ml 1N-Salzsäure / 50 ml Essigsäureethylester geschüttelt, die organische Phase abgetrennt, mit Natriumsulfat getrocknet und filtriert. Das Filtrat wird im Wasserstrahlvakuum eingeengt, in Essigsäureethylester gelöst, mit 5%iger wässriger Dinatriumhydrogenphosphat-Lösung gewaschen, mit Natriumsulfat getrocknet und filtriert. Das Filtrat wird im Wasserstrahlvakuum eingeengt, der Rückstand mit Petrolether verrührt und das Lösungsmittel im Wasserstrahlvakuum sorgfältig abdestilliert.

Man erhält 0,3 g (57% der Theorie) 4-(3,6-Dihydro-2,6-dioxo-3-methyl-4-trifluormethyl-1(2H)-pyrimidin-1-yl)-5-fluor-2-(4-methoxy-phenoxy)-benzonitril vom Schmelzpunkt 62°C.

Analog zu Herstellungsbeispiel 1 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

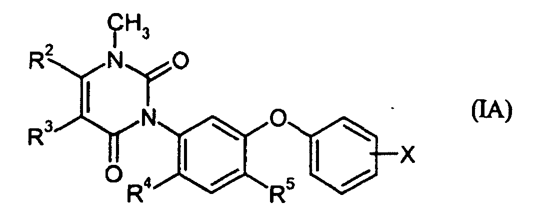

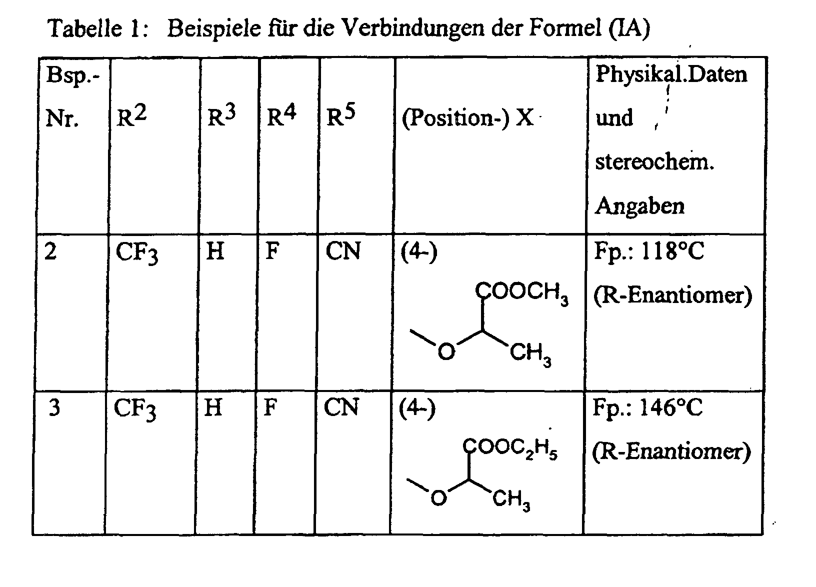

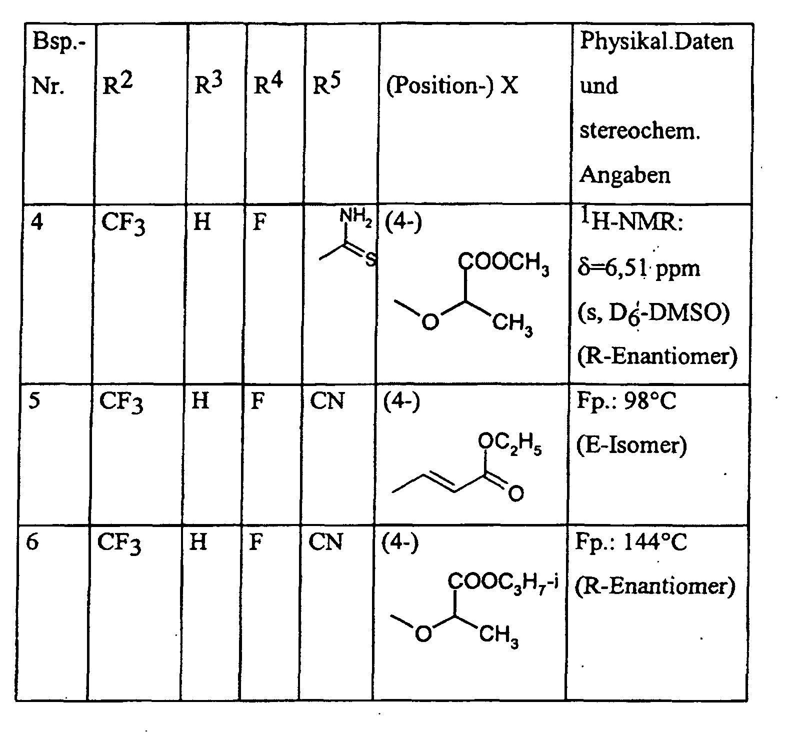

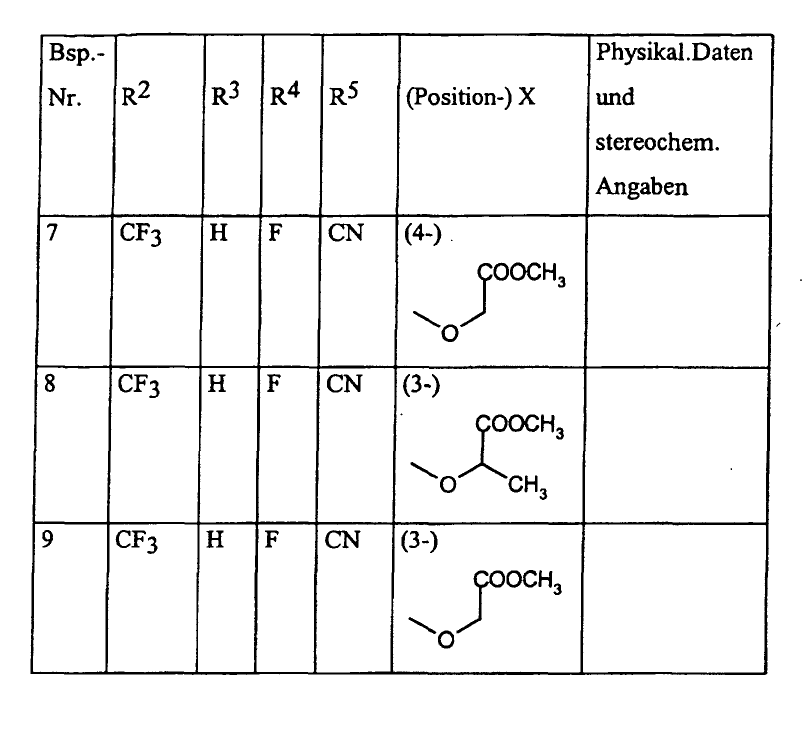

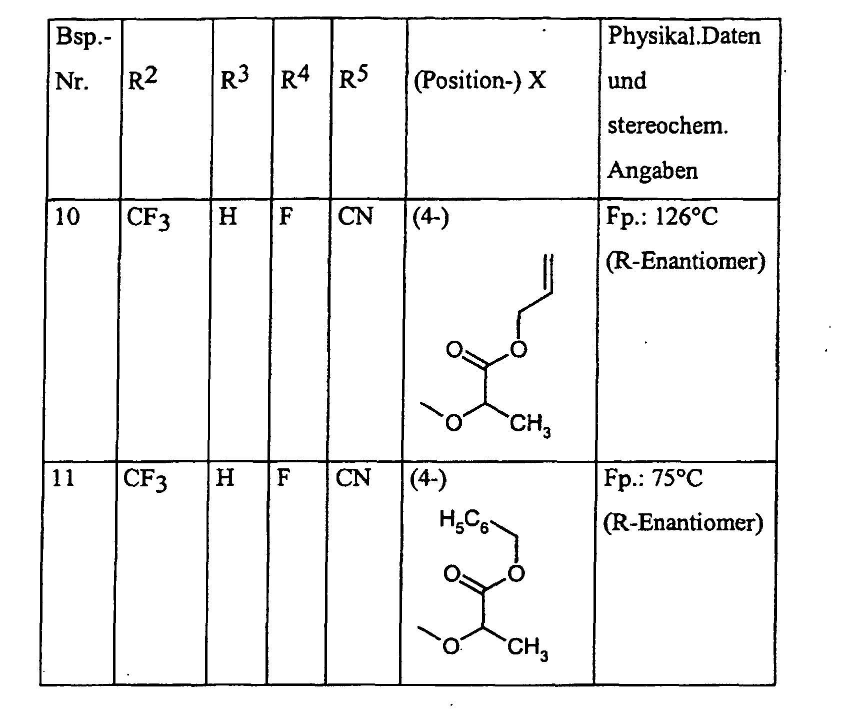

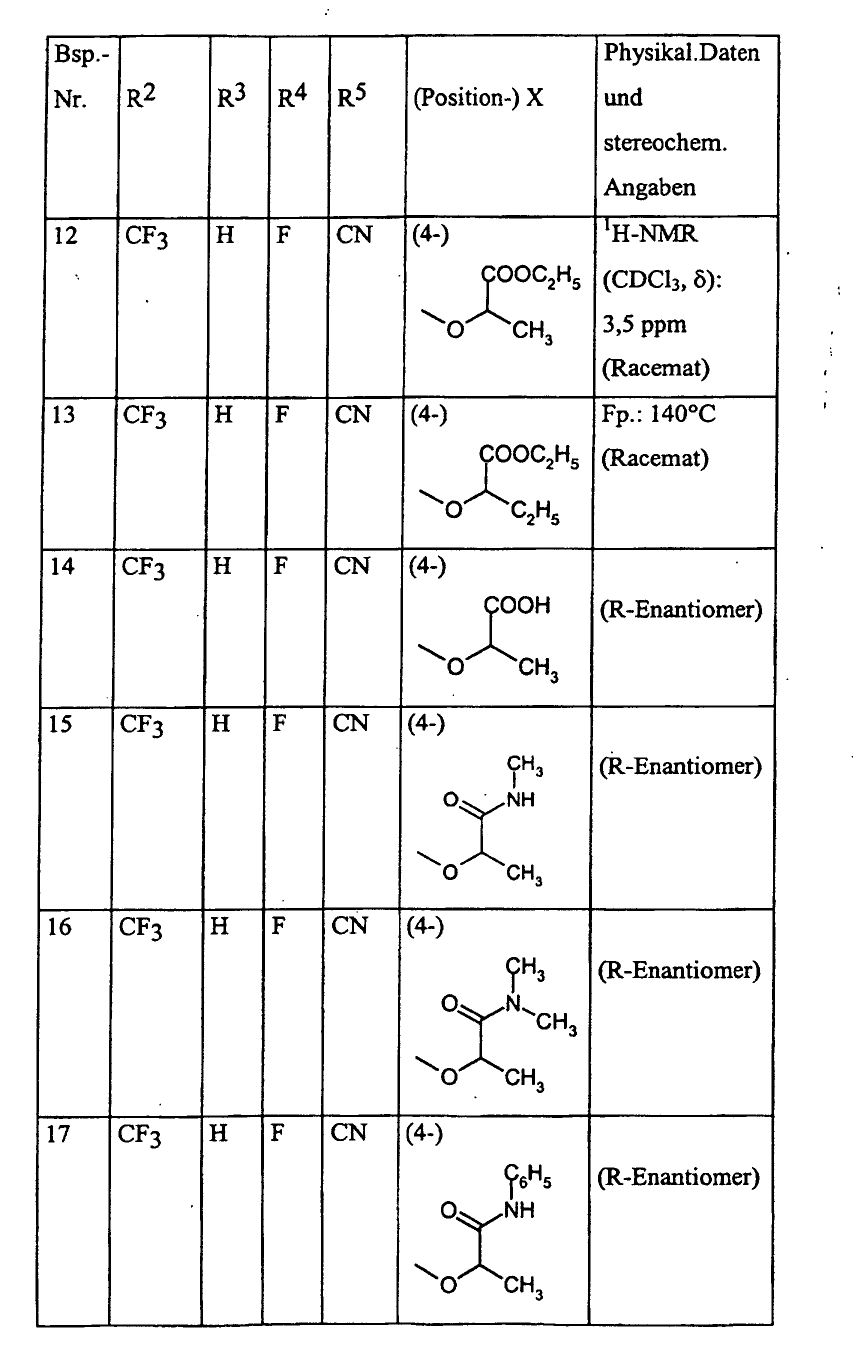

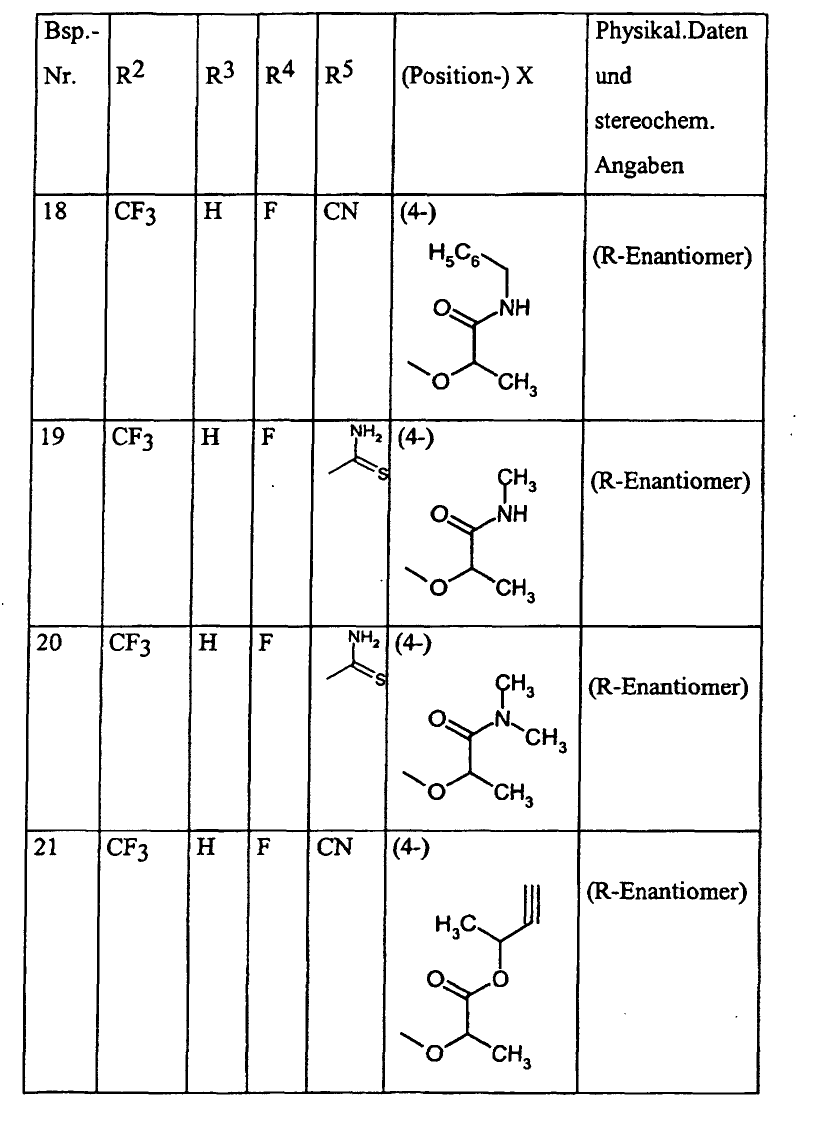

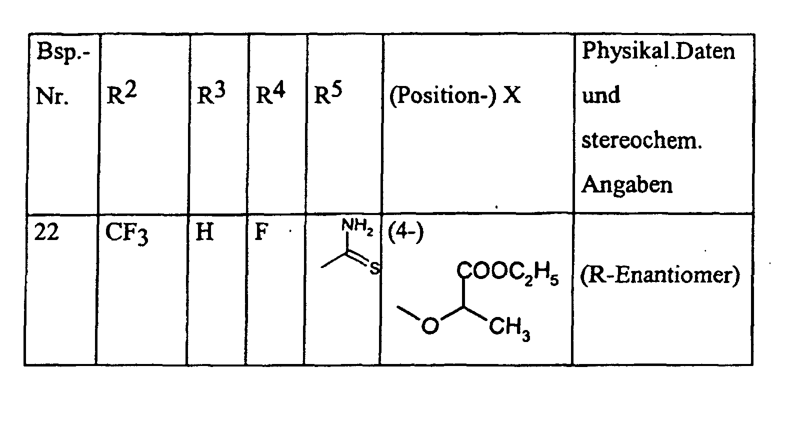

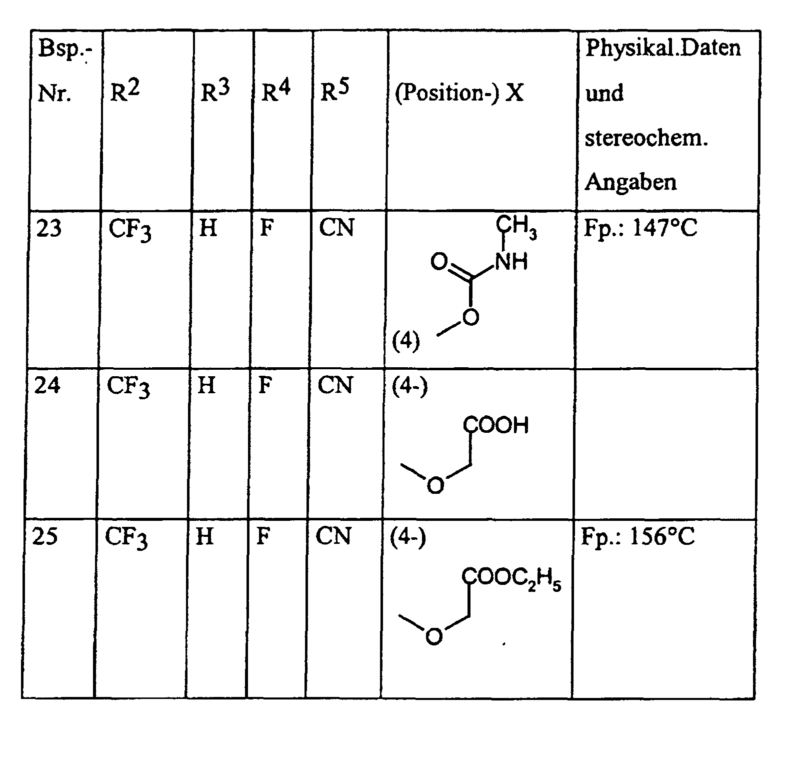

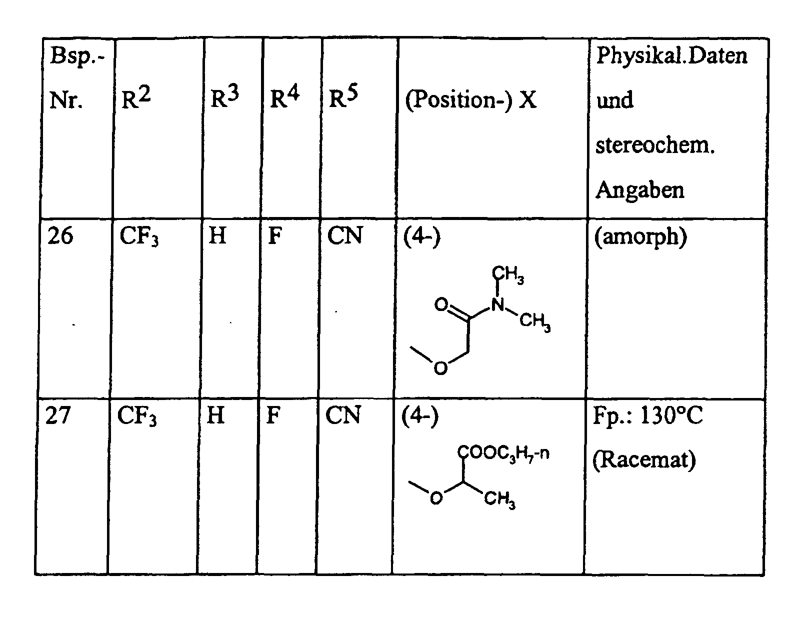

### Ausgangsstoffe der Formel (IX):

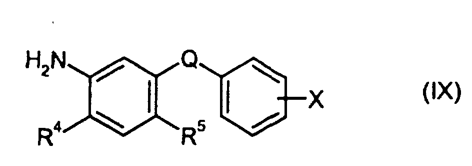

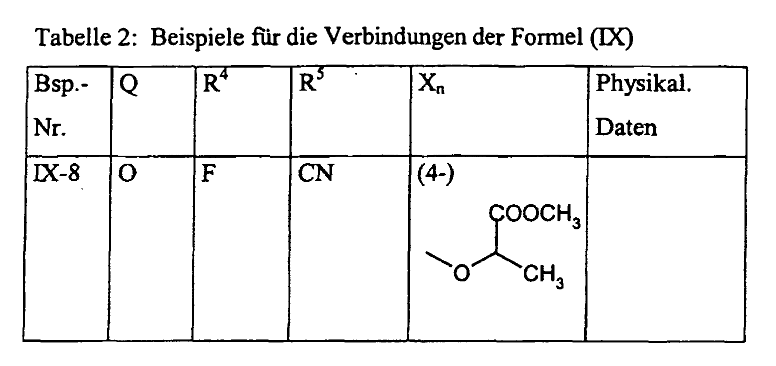

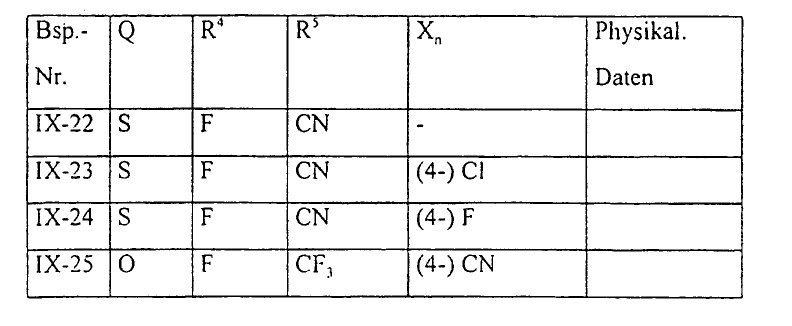

### Anwendungsbeispiele:

### Beispiel A

Pre-emergence-Test

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden so mit der Wirkstoffzubereitung besprüht, daß die jeweils gewünschte Wirkstoffmenge pro Flächeneinheit ausgebracht wird. Die Konzentration der Spritzbrühe wird so gewählt, daß in 1000 Liter Wasser pro Hektar die jeweils gewünschte Wirkstoffmenge ausgebracht wird.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

In diesem Test zeigen beispielsweise die Verbindungen gemäß Herstellungsbeispiel 2 und 3 starke Wirkung gegen Unkräuter.

### Beispiel B

### Post-emergence-Test

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

In diesem Test zeigen beispielsweise die Verbindungen gemäß Herstellungsbeispiel 2 und 3 starke Wirkung gegen Unkräuter.

## Patentansprüche

1. Substituierte Phenyluracile der allgemeinen Formel (IA), 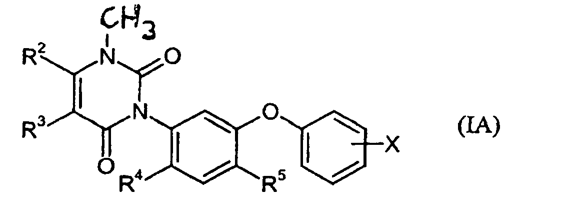 in welcher
R² für Trifluormethyl, Chlordifluormethyl, Difluormethyl oder Pentafluorethyl steht,
R³ für Wasserstoff, Chlor oder Methyl steht,
R⁴ für Wasserstoff, Fluor oder Chlor steht,
R⁵ für Cyano oder Thiocarbamoyl steht, und
X für jeweils durch Carboxy, Methoxy, Ethoxy, n- oder i-Propoxy, Methoxycarbonyl, Ethoxycarbonyl, n- oder i-Propoxy-carbonyl, Allyloxycarbonyl, Propargyloxycarbonyl, 1-Buten-3-yl-oxy-carbonyl, 2-Buten-4-yl-oxycarbonyl, Propargyloxycarbonyl, 1-Butin-3-yl-oxy-carbonyl, 2-Butin-4-yl-oxy-carbonyl, Methylaminocarbonyl, Ethylaminocarbonyl, n- oder i-Propylamino-carbonyl, Dimethylaminocarbonyl, Diethylamino-carbonyl, Phenoxycarbonyl, Benzyloxycarbonyl, Phenylaminocarbonyl oder Benzylaminocarbonyl substituiertes Methyl, Ethyl, Methoxy, Ethoxy, Methylthio oder Ethylthio, oder für durch Methoxycarbonyl oder Ethoxycarbonyl substiutiertes Ethenyl steht.

2. Substituierte Phenyluracile gemäß Anspruch 1, **dadurch gekennzeichnet, dass**
R² für Trifluormethyl steht,
R³ für Wasserstoff steht,
R⁴ für Fluor steht,
R⁵ für Cyano steht, und
X für 4-Carboxymethoxy, 4-Methoxycarbonylmethoxy, 4-Ethoxycarbonylmethoxy, 4-n-Propoxycarbonylmethoxy, 4-i-Propoxycarbonylmethoxy, 4-(1-Carboxy-ethoxy), 4-(1-(Methoxycarbonyl)-ethoxy), 4-(1-(Ethoxycarbonyl)-ethoxy), 4-(1-(n-Propoxycarbonyl)-ethoxy), 4-(1-(i-Propoxycarbonyl)-ethoxy), 4-(Allyloxycarbonylmethoxy), 4-(1-(Allyloxycarbonyl)-ethoxy), 4-(Propargyloxycarbonylmethoxy), 4-(1-(Propargyloxycarbonyl)-ethoxy), 4-(Benzyloxycarbonylmethoxy), 4-(1-(Benzyloxycarbonyl)-ethoxy), 4-(Aminocarbonylmethoxy), 4-(Methylaminocarbonylmethoxy), 4-(Ethylaminocarbonylmethoxy), 4-(n-Propylaminocarbonylmethoxy), 4-(i-Propyl-aminocarbonylmethoxy), 4-(Dimethylaminocarbonylmethoxy), 4-(1-(Methylaminocarbonyl)-ethoxy), 4-(1-(Ethylaminocarbonyl)-ethoxy), 4-(1-(n-Propylaminocarbonyl)-ethoxy), 4-(1-(i-Propylaminocarbonyl)-ethoxy), 4-(1-(Dimethylaminocarbonyl)-ethoxy), 4-(2-Methoxycarbonyl-ethenyl), 4-(2-Ethoxycarbonyl-ethenyl).

3. Substituierte Phenyluracile gemäß Anspruch 1, **dadurch gekennzeichnet, dass**
R² für Trifluormethyl steht,
R³ für Wasserstoff steht,
R⁴ für Fluor steht,
R⁵ für Thiocarbamoyl steht, und
X für 4-Carboxymethoxy, 4-Methoxycarbonylmethoxy, 4-Ethoxycarbonylmethoxy, 4-n-Propoxycarbonylmethoxy, 4-i-Propoxycarbonylmethoxy, 4-(1-Carboxy-ethoxy), 4-(1-(Methoxycarbonyl)-ethoxy), 4-(1-(Ethoxycarbonyl)-ethoxy), 4-(1-(n-Propoxycarbonyl)-ethoxy), 4-(1-(i-Propoxycarbonyl)-ethoxy), 4-(Allyloxycarbonylmethoxy), 4-(1-(Allyloxycarbonyl)-ethoxy), 4-(Propargyloxycarbonylmethoxy), 4-(1-(Propargyloxycarbonyl)-ethoxy), 4-(Benzyloxycarbonylmethoxy), 4-(1-(Benzyloxycarbonyl)-ethoxy), 4-(Aminocarbonylmethoxy), 4-(Methylaminocarbonylmethoxy), 4-(Ethylaminocarbonylmethoxy), 4-(n-Propylaminocarbonylmethoxy), 4-(i-Propyl-aminocarbonylmethoxy), 4-(Dimethylaminocarbonylmethoxy), 4-(1-(Methylaminocarbonyl)-ethoxy), 4-(1-(Ethylaminocarbonyl)-ethoxy), 4-(1-(n-Propylaminocarbonyl)-ethoxy), 4-(1-(i-Propylaminocarbonyl)-ethoxy), 4-(1-(Dimethylaminocarbonyl)-ethoxy), 4-(2-Methoxycarbonyl-ethenyl), 4-(2-Ethoxycarbonyl-ethenyl) steht.

4. Verfahren zum Herstellen von substituierten Phenyluracilen gemäß einem der Ansprüche 1 bis 3, **dadurch gekennzeichnet, dass** man
(a) Halogenophenyluracile der allgemeinen Formel (II) 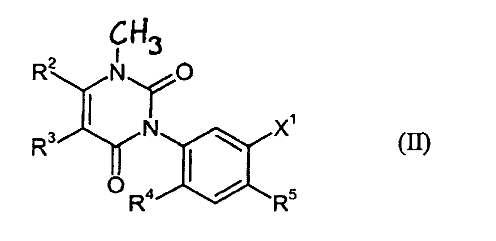 in welcher
R², R³, R⁴ und R⁵ die in einem der Ansprüche 1 bis 3 angegebene Bedeutung haben und
X¹ für Halogen steht,
mit Arylverbindungen der allgemeinen Formel (III) 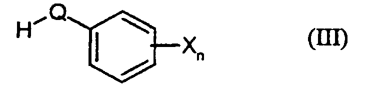 in welcher
n, Q und X die in einem der Ansprüche 1 bis 3 angegebene Bedeutung haben,
- oder mit Metallsalzen von Verbindungen der allgemeinen Formel (III) -
gegebenenfalls in Gegenwart eines Reaktionshilfsmittels und gegebenenfalls in Gegenwart eines Verdünnungsmittels umsetzt,
oder dass man
(b) Aminoalkensäureester der allgemeinen Formel (IV) 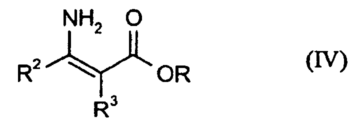 in welcher
R² und R³ die in einem der Ansprüche 1 bis 3 angegebene Bedeutung haben und
R für C₁-C₄-Alkyl, Phenyl oder Benzyl steht,
mit Arylisocyanaten der allgemeinen Formel (V) 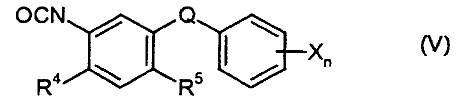 in welcher
n, Q, R⁴, R⁵ und X die in einem der Ansprüche 1 bis 3 angegebene Bedeutung haben,
oder mit Arylurethanen (Arylcarbamaten) der allgemeinen Formel (VI) 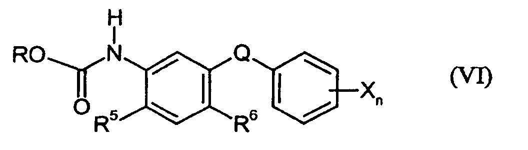 in welcher
n, Q, R⁵, R⁶ und X die in einem der Ansprüche 1 bis 3 angegebene Bedeutung haben und
R für C₁-C₄-Alkyl, Phenyl oder Benzyl steht,
gegebenenfalls in Gegenwart eines Reaktionshilfsmittels und gegebenenfalls in Gegenwart eines Verdünnungsmittels umsetzt,
oder dass man
(c) N-Aryl-1-alkoxycarbonylamino-maleinimide der allgemeinen Formel (VII) 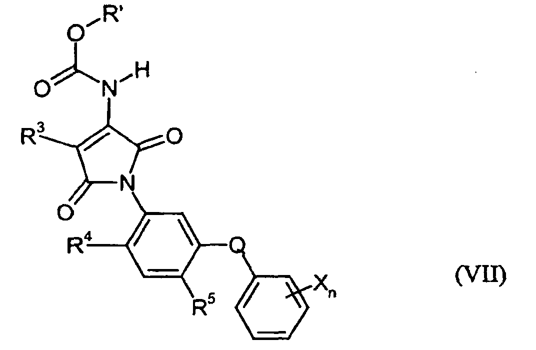 in welcher
n, Q, R³, R⁴, R⁵ und X die in einem der Ansprüche 1 bis 3 angegebene Bedeutung haben und
R' für C₁-C₄-Alkyl steht,
mit einem Metallhydroxid in Gegenwart von Wasser und gegebenenfalls in Gegenwart eines organischen Lösungsmittels umsetzt,
oder dass man
(d) substituierte Phenyluracile der allgemeinen Formel (Ia) 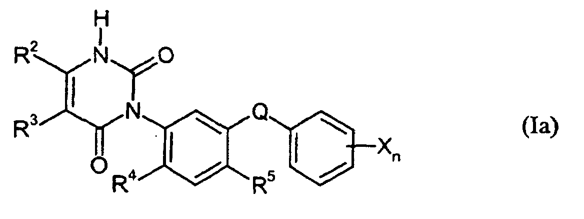
in welcher
n, Q, R², R³, R⁴, R⁵ und X die in einem der Ansprüche 1 bis 3 angegebene Bedeutung haben,
mit 1-Aminooxy-2,4-dinitro-benzol oder mit Alkylierungsmitteln der allgemeinen Formel (VIII)
X²-A¹ (VIII)
in welcher
A¹ für gegebenenfalls durch Cyano, Fluor, Chlor, Methoxy oder Ethoxy substituiertes Methyl, Ethyl, n- oder i-Propyl steht und
X² für Chlor, Brom, Iod, Methylsulfonyloxy oder Ethylsulfonyloxy steht,
gegebenenfalls in Gegenwart eines Reaktionshilfsmitteis und gegebenenfalls in Gegenwart eines Verdünnungsmittels umsetzt,
und gegebenenfalls im Anschluß daran im Rahmen der Substituentendefinition auf übliche Weise elektrophile oder nucleophile bzw. Oxidations- oder Reduktionsreaktionen durchführt.

5. Verwendung von mindestens einem substituierten Phenyluracil gemäß einem der Ansprüche 1 bis 3 zur Bekämpfung von unerwünschten Pflanzen.

6. Herbizide Mittel, **gekennzeichnet durch** den Gehalt an mindestens einem substituierten Phenyluracil gemäß einem der Ansprüche 1 bis 3.

## Claims

1. A substituted phenyluracil of the formula (IA) 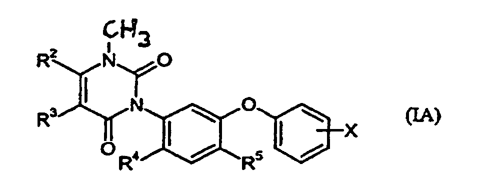 in which
R² represents trifluoromethyl, chlorodifluoromethyl, difluoromethyl or pentafluoroethyl,
R³ represents hydrogen, chlorine or methyl,
R⁴ represents hydrogen, fluorine or chlorine,
R⁵ represents cyano or thiocarbamoyl, and
X represents methyl, ethyl, methoxy, ethoxy, methylthio or ethylthio, each of which is substituted by carboxyl, methoxy, ethoxy, n- or i-propoxy, methoxycarbonyl, ethoxycarbonyl, n-or i-propoxycarbonyl, allyloxycarbonyl, propargyloxycarbonyl, 1-buten-3-yloxycarbonyl, 2-buten-4-yloxycarbonyl, propargyloxycarbonyl, 1-butin-3-yloxycarbonyl, 2-butin-4-yloxycarbonyl, methylaminocarbonyl, ethylaminocarbonyl, n- or i-propylaminocarbonyl, dimethylaminocarbonyl, diethylaminocarbonyl, phenoxycarbonyl, benzyloxycarbonyl, phenylaminocarbonyl or benzylaminocarbonyl, or represents ethenyl which is substituted by methoxycarbonyl or ethoxycarbonyl.

2. The substituted phenyluracil as claimed in claim 1, wherein
R² represents trifluoromethyl,
R³ represents hydrogen,
R⁴ represents fluorine,
R⁵ represents cyano, and
X represents 4-carboxymethoxy, 4-methoxycarbonylmethoxy, 4-ethoxycarbonylmethoxy, 4-n-propoxycarbonylmethoxy, 4-i-propoxycarbonylmethoxy, 4-(1-carboxyethoxy), 4-(1-methoxycarbonyl)-ethoxy), 4-(1-ethoxycarbonyl)ethoxy), 4-(1-(n-propoxycarbonyl)ethoxy), 4-(1-(i-propoxycarbonyl)ethoxy), 4-(allyloxycarbonylmethoxy), 4-(1-(allyloxycarbonyl)ethoxy), 4-(propargyloxycarbonylmethoxy), 4-(1-propargyloxycarbonyl)ethoxy), 4-(benzyloxycarbonylmethoxy), 4-(1-benzyloxycarbonyl)-ethoxy), 4-(aminocarbonylmethoxy), 4-(methylaminocarbonylmethoxy), 4-(ethylaminocarbonylmethoxy), 4-(n-propylaminocarbonylmethoxy), 4-(i-propylaminocarbonylmethoxy), 4-(dimethylaminocarbonylmethoxy) 4-(1-(methylaminocarbonyl)ethoxy), 4-(1-(ethylaminocarbonyl)-ethoxy), 4-(1-(n-propylaminocarbonyl)ethoxy), 4-(1-(i-propylaminocarbonyl)ethoxy), 4-(1-(dimethylaminocarbonyl)ethoxy), 4-(2-methoxycarbonylethenyl), 4-(2-ethoxycarbonylethenyl).

3. The substituted phenyl uracil as claimed in claim 1, wherein
R² represents trifluoromethyl,
R³ represents hydrogen,
R⁴ represents fluorine,
R⁵ represents thiocarbamoyl, and
X represents 4-carboxymethoxy, 4-methoxycarbonylmethoxy, 4-ethoxycarbonylmethoxy, 4-n-propoxycarbonylmethoxy, 4-i-propoxycarbonylmethoxy, 4-(1-carboxyethoxy), 4-(1-methoxycarbonyl)-ethoxy), 4-(1-ethoxycarbonyl)ethoxy), 4-(1-(n-propoxycarbonyl)ethoxy, 4-(1-(i-propoxycarbonyl)ethoxy), 4-(allyloxycarbonylmethoxy), 4-(1-(allyloxycarbonyl)-ethoxy), 4-(propargyloxycarbonylmethoxy), 4-(1-propargyloxycarbonyl)ethoxy), 4-(benzyloxycarbonylmethoxy), 4-(1-benzyloxycarbonyl)-ethoxy), 4-(aminocarbonylmethoxy), 4-(methylaminocarbonylmethoxy), 4-(ethylaminocarbonylmethoxy), 4-(n-propylaminocarbonylmethoxy), 4-(i-propylaminocarbonylmethoxy), 4-(dimethylaminocarbonylmethoxy) 4-(1-(methylaminocarbonyl)ethoxy), 4-(1-(ethylaminocarbonyl)-ethoxy), 4-(1-(n-propylaminocarbonyl)ethoxy), 4-(1-(i-propylaminocarbonyl)ethoxy), 4-(1-(dimethylaminocarbonyl)ethoxy), 4-(2-methoxycarbonylethenyl), 4-(2-ethoxycarbonylethenyl).

4. A process for the preparation of substituted phenyluracils as claimed in any of claims 1 to 3, wherein
(a) halogenophenyluracils of the formula (II) 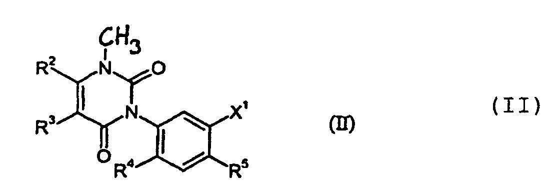 in which
R², R³, R⁴ and R⁵ have the meaning given in any of claims 1 to 3 and
X¹ represents halogen
are reacted with aryl compounds of the formula (III) 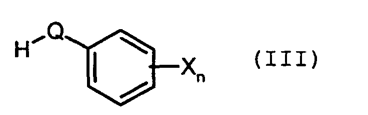 in which
n, Q and X have the meaning given in any of claims 1 to 3
- or with metal salts of compounds of the formula (III) -
if appropriate in the presence of a reaction auxiliary and if appropriate in the presence of a diluent,
or wherein
(b) aminoalkenoic esters of the formula (IV) 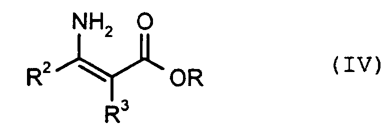 in which
R² and R³ have the meaning given in any of claims 1 to 3 and
R represents C₁-C₄-alkyl, phenyl or benzyl,
are reacted with aryl isocyanates of the formula (V) 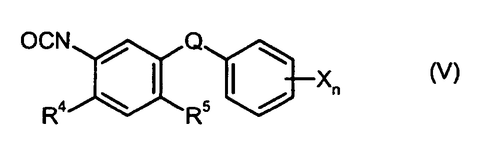 in which
n, Q, R⁴, R⁵ and X have the meaning given in any of claims 1 to 3,
or with arylurethanes (aryl carbamates) of the formula (VI) 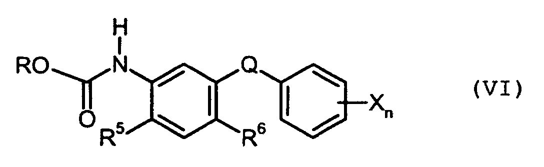 in which
n, Q, R⁵, R⁶ and X have the meaning given in any of claims 1 to 3 and
R represents C₁-C₄-alkyl, phenyl or benzyl,
if appropriate in the presence of a reaction auxiliary and if appropriate in the presence of a diluent,
or wherein
(c) N-aryl-1-alkoxycarbonylaminomaleimides of the formula (VII) 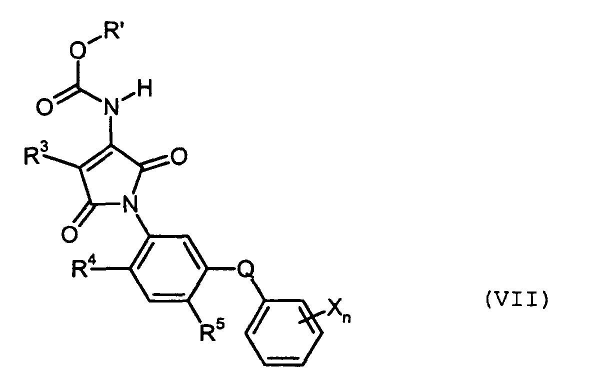 in which
n, Q, R³ , R⁴, R⁵ and X have the meaning given in any of claims 1 to 3 and
R' represents C₁-C₄-alkyl
are reacted with a metal hydroxide in the presence of water and if appropriate in the presence of an organic solvent,
or wherein
(d) substituted phenyluracils of the formula (Ia) 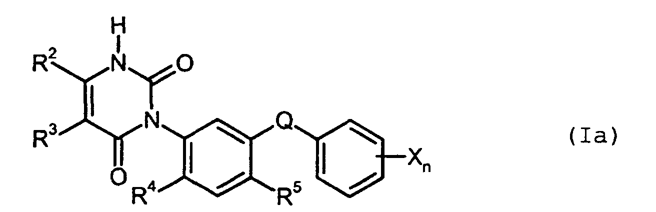
in which
n, Q, R², R³, R⁴, R⁵ and X have the meaning given in any of claims 1 to 3
are reacted with 1-aminooxy-2,4-dinitrobenzene or with alkylating agents of the formula (VIII)
X²-A¹ (VIII)
in which
A¹ represents methyl, ethyl, n- or i-propyl, each of which is optionally substituted by cyano, fluorine, chlorine, methoxy or ethoxy, and
X² represents chlorine, bromine, iodine, methysulfonyloxy or ethylsulfonyloxy,
if appropriate in the presence of a reaction auxiliary and if appropriate in the presence of a diluent,
and, if appropriate, electrophilic or nucleophilic or oxidation or reduction reactions are subsequently carried out in the customary manner within the scope of the definition of the substituents.

5. The use of at least one substituted phenyluracil as claimed in any of claims 1 to 3 for controlling undesired plants.

6. A herbicidal composition, which comprises at least one substituted phenyluracil as claimed in any of claims 1 to 3.

## Revendications

1. Phényluraciles substitués de formule générale (IA) 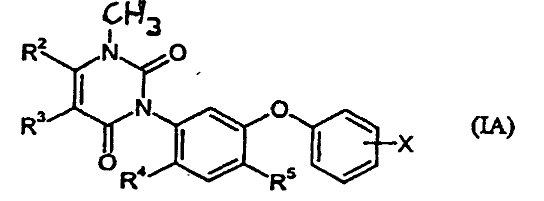 dans laquelle
R² représente un groupe trifluorométhyle, chlorodifluorométhyle, difluorométhyle ou pentafluoroéthyle,
R³ représente un atome d'hydrogène, un atome de chlore ou un groupe méthyle,
R⁴ représente un atome d'hydrogène, un atome de fluor ou de chlore,
R⁵ représente un groupe cyano ou thiocarbamoyle, et
X représente un groupe méthyle, éthyle, méthoxy, éthoxy, méthylthio ou éthylthio, chacun substitué par un substituant carboxy, méthoxy, éthoxy, n- ou i-propoxy, méthoxycarbonyle, éthoxycarbonyle, n- ou i-propoxycarbonyle, allyloxycarbonyle, propargyloxycarbonyle, 1-butén-3-yl-oxy-carbonyle, 2-butén-4-yl-oxy-carbonyle, propargyloxycarbonyle, 1-butyn-3-yl-oxy-carbonyle, 2-butyn-4-yl-oxy-carbonyle, méthylaminocarbonyle, éthylaminocarbonyle, n-ou i-propyl-amino-carbonyle, diméthylaminocarbonyle, diéthylaminocarbonyle, phénoxycarbonyle, benzyloxycarbonyle, phénylaminocarbonyle ou benzylaminocarbonyle, ou un groupe éthényle substitué par un substituant méthoxycarbonyle ou éthoxycarbonyle.

2. Phényluraciles substitués selon la revendication 1, **caractérisés en ce que**
R² représente un groupe trifluorométhyle,
R³ représente un atome d'hydrogène,
R⁴ représente un atome de fluor,
R⁵ représente un groupe cyano, et
X représente un groupe 4-carboxyméthoxy, 4-méthoxycarbonylméthoxy, 4-éthoxycarbonylméthoxy, 4-n-propoxycarbonylméthoxy, 4-i-propoxycarbonylméthoxy, 4-(1-carboxy-éthoxy), 4-(1-(méthoxycarbonyl)-éthoxy), 4-(1-(éthoxycarbonyl)-éthoxy), 4-(1-(n-propoxycarbonyl)-éthoxy), 4-(1-(i-propoxycarbonyl)-éthoxy), 4-(allyloxycarbonylméthoxy), 4-(1-(allyloxycarbonyl)-éthoxy), 4-(propargyloxycarbonylméthoxy), 4-(1-(propargyloxycarbonyl)-éthoxy), 4-(benzyloxycarbonylméthoxy), 4-(1-(benzyloxycarbonyl)-éthoxy), 4-(aminocarbonylméthoxy), 4-(méthylaminocarbonylméthoxy), 4-(éthylaminocarbonylméthoxy), 4-(n-propylaminocarbonylméthoxy), 4-(i-propyl-aminocarbonylméthoxy), 4-(diméthylaminocarbonylméthoxy), 4-(1-(méthylaminocarbonyl)-éthoxy), 4-(1-(éthylaminocarbonyl)-éthoxy), 4-(1-(n-propylaminocarbonyl)-éthoxy), 4-(1-(i-propylaminocarbonyl)-éthoxy), 4-(1-(diméthylaminocarbonyl)-éthoxy), 4-(2-méthoxycarbonyléthényle), 4-(2-éthoxycarbonyl-éthényle).

3. Phényluraciles substitués selon la revendication 1, **caractérisés en ce que**
R² représente un groupe trifluorométhyle,
R³ représente un atome d'hydrogène,
R⁴ représente un atome de fluor,
R⁵ représente un groupe thiocarbamoyle, et
X représente un groupe 4-carboxyméthoxy, 4-méthoxycarbonylméthoxy, 4-éthoxycarbonylméthoxy, 4-n-propoxycarbonylméthoxy, 4-i-propoxycarbonylméthoxy, 4-(1-carboxy-éthoxy), 4-(1-(méthoxycarbonyl)-éthoxy), 4-(1-(éthoxycarbonyl)-éthoxy), 4-(1-(n-propoxycarbonyl)-éthoxy), 4-(1-(i-propoxy-carbonyl)-éthoxy), 4-(allyloxycarbonylméthoxy), 4-(1-(allyloxycarbonyl)-éthoxy), 4-(propargyloxycarbonylméthoxy), 4-(1-(propargyloxycarbonyl)-éthoxy), 4-(benzyloxycarbonylméthoxy), 4-(1-(benzyloxycarbonyl)-éthoxy), 4-(aminocarbonylméthoxy), 4-(méthylaminocarbonylméthoxy), 4-(éthylaminocarbonylméthoxy), 4-(n-propylaminocarbonylméthoxy), 4-(i-propyl-aminocarbonylméthoxy), 4-(diméthylaminocarbonylméthoxy), 4-(1-(méthylaminocarbonyl)-éthoxy), 4-(1-(éthylaminocarbonyl)-éthoxy), 4-(1-(n-propylaminocarbonyl)-éthoxy), 4-(1-(i-propylaminocarbonyl)-éthoxy), 4-(1-(diméthylaminocarbonyl)-éthoxy), 4-(2-méthoxycarbonyléthényle), 4-(2-éthoxycarbonyl-éthényle).

4. Procédé pour la préparation de phényluraciles substitués selon l'une des revendications 1 à 3, **caractérisé en ce qu'**on fait réagir
(a) des halogénophényluraciles de formule générale (II) 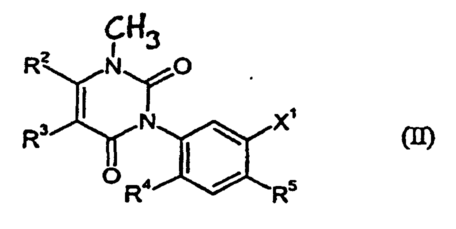 dans laquelle
R², R³, R⁴ et R⁵ possèdent l'une des significations données dans les revendications 1 à 3 et
X¹ représente un atome d'halogène,
sur des composés aryliques de formule générale (III) 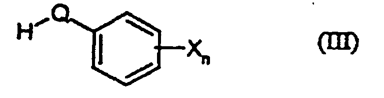 dans laquelle
n, Q et X possèdent la signification donnée dans l'une des revendications 1 à 3,
- ou sur des sels métalliques de composés de formule générale (III) -
éventuellement en présence d'un agent de réaction auxiliaire et éventuellement en présence d'un diluant,
ou **en ce qu'**on fait réagir
(b) des esters d'acides aminoalcéniques de formule générale (IV) 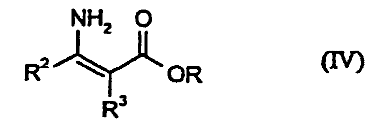 dans laquelle
R² et R³ possèdent la signification donnée dans l'une des revendications 1 à 3, et
R représente un groupe alkyle en C₁-C₄, phényle ou benzyle,
sur des arylisocyanates de formule générale (V) 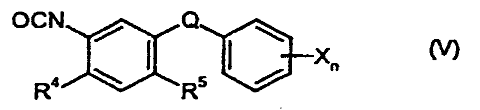 dans laquelle
n, Q, R⁴, R⁵ et X possèdent la signification donnée dans l'une des revendications 1 à 3,
ou sur des aryluréthannes (arylcarbamates) de formule générale (VI) 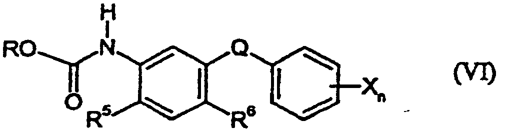 dans laquelle
n, Q, R⁵, R⁶ et X possèdent la signification donnée dans l'une des revendications 1 à 3, et
R représente un groupe alkyle en C₁-C₄, phényle ou benzyle,
éventuellement en présence d'un agent de réaction auxiliaire et éventuellement en présence d'un diluant,
ou **en ce qu'**on fait réagir
c) un N-aryl-1-alkoxycarbonylamino-maléimide de formule générale (VII) 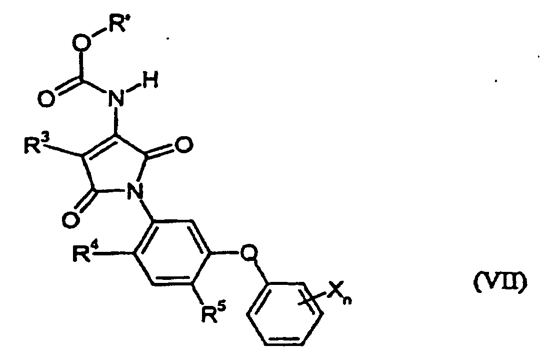 dans laquelle
n, Q, R³, R⁴, R⁵ et X possèdent la signification donnée dans l'une des revendications 1 à 3,
R' représente un groupe alkyle en C₁-C₄,
sur un hydroxyde métallique en présence d'eau et éventuellement en présence d'un solvant organique, ou **en ce qu'**on fait réagir
(d) des phényluraciles substitués de formule générale (Ia) 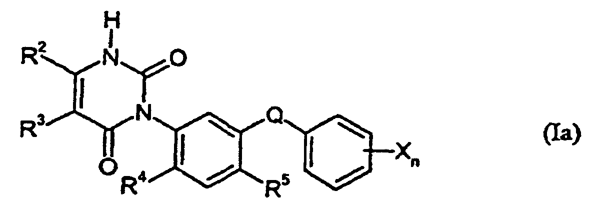
dans laquelle
n, Q, R², R³, R⁴, R⁵ et X possèdent la signification donnée dans l'une des revendications 1 à 3,
sur un 1-aminooxy-2,4-dinitro-benzène ou sur des agents alkylants de formule générale (VIII)
X²-A¹ (VIII)
dans laquelle
A¹ représente un groupe méthyle, éthyle, n- ou i-propyle, éventuellement substitué par un substituant cyano, fluor, chlore, méthoxy ou éthoxy, et
X² représente un atome de chlore, de brome, d'iode, un groupe méthylsulfonyloxy ou éthylsulfonyloxy,
éventuellement en présence d'un agent de réaction auxiliaire et éventuellement en présence d'un diluant,
et éventuellement par la suite, on met en oeuvre de façon usuelle, dans le cadre de la définition des substituants, des réactions électrophiles ou nucléophiles ou d'oxydation et de réduction.

5. Utilisation d'au moins un phényluracile substitué selon l'une des revendications 1 à 3 dans la lutte contre des plantes indésirables.

6. Agents herbicides, **caractérisés par** la teneur en au moins un phényluracile substitué selon l'une des revendications 1 à 3.
